(19) **Europäisches Patentamt / European Patent Office / Office européen des brevets**

(11) **EP 1 339 481 B1**

(12) **FASCICULE DE BREVET EUROPEEN**

(45) Date de publication et mention
de la délivrance du brevet:
**10.03.2010 Bulletin 2010/10**

(51) Int Cl.:
**B01D 65/02** (2006.01)   **A61L 2/08** (2006.01)
**A61M 1/16** (2006.01)

(21) Numéro de dépôt: **01999421.9**

(22) Date de dépôt: **03.12.2001**

(86) Numéro de dépôt international:
**PCT/IB2001/002297**

(87) Numéro de publication internationale:
**WO 2002/045830 (13.06.2002 Gazette 2002/24)**

(54) **Utilisation d'une solution aqueuse de glycérol pour préserver les performances de membranes semi-perméables humides destinées au traitement du sang ou du plasma**

Verwendung einer wässrigen Glyzerol Lösung zur Beibehaltung der Leistungen von teildurchlässigen feuchten Membranen für die Behandlung von Blut und Plasma

Use of a glycerol aqueous solution for maintaining the performances of semi-permeable wet membranes devoted to the treatment of blood or plasma

(84) Etats contractants désignés:
**AT BE CH CY DE DK ES FI FR GB GR IE IT LI LU MC NL PT SE TR**

(30) Priorité: **08.12.2000 FR 0016007**

(43) Date de publication de la demande:
**03.09.2003 Bulletin 2003/36**

(73) Titulaire: **Gambro Industries SAS**
**69330 Meyzieu (FR)**

(72) Inventeur: **THOMAS, Michel**
**F-69360 Serezin du Rhone (FR)**

(74) Mandataire: **Ponzellini, Gianmarco et al**
**Gambro Legal and Intellectual Property Dept.**
**P.O. Box 98**
**41037 Mirandola (IT)**

(56) Documents cités:
**EP-A- 0 315 420   EP-A- 0 801 953**
**US-A- 4 609 728**

• **DATABASE WPI Week 197744 Derwent Publications Ltd., London, GB; AN 1977-78163Y XP002175645 & JP 50 128773 A ((TORA) TORAY IND INC), 11 octobre 1975 (1975-10-11)**
• **PATENT ABSTRACTS OF JAPAN vol. 0176, no. 22 (C-1130), 17 novembre 1993 (1993-11-17) & JP 05 192397 A (NIKKISO CO LTD), 3 août 1993 (1993-08-03)**

**Description**

La présente invention concerne un procédé de traitement d'appareils pour le traitement du sang ou du plasma par circulation extracorporelle comprenant une membrane semi-perméable humide.

Par membrane semi-perméable humide, on entend, dans l'ensemble de ce texte, une membrane semi-perméable plane ou un faisceau de fibres creuses semi-perméables, qui contient au moins 40% en poids d'eau, rapporté au poids de la membrane semi-perméable. Egalement, par appareil, on entend, dans l'ensemble de ce texte, un appareil pour le traitement du sang ou du plasma par circulation extracorporelle qui comprend, d'une manière générale, deux compartiments séparés par une membrane semi-perméable, munis chacun de deux accès, un premier compartiment étant destiné à la circulation du sang ou du plasma du patient, et un second compartiment étant destiné à la circulation de liquide usé. Les deux compartiments de l'appareil sont, en outre, séparés par la masse d'empotage, à base d'une composition adhésive appropriée, destinée à former, selon le cas :

- une cloison cylindrique de séparation des deux compartiments d'un appareil dont la membrane est constituée par un faisceau de fibres creuses ;

- ou un joint étanche dans un appareil comprenant une membrane plane.

Des appareils pour le traitement du sang ou du plasma par circulation extracorporelle sont utilisés dans diverses applications médicales ou paramédicales telles que : traitement de l'insuffisance rénale par dialyse ou hémofiltration, plasmaphérèse et aphérèse à visée thérapeutique et non thérapeutique, oxygénation du sang, immunoépuration, etc.

La présente invention concerne également l'utilisation d'une solution aqueuse de glycérol pour limiter les risques de fuite et les variations des performances des structures en matériau polymère, semi-perméables et humides, en particulier limiter les variations de la perméabilité hydraulique des membranes semi-perméables humides, dans un intervalle de valeurs acceptables après que les appareils comprenant ces membranes ont été soumis à une température inférieure à 0° C pendant une période pouvant aller de un jour à plus d'un mois. Par exemple, ceci peut se produire durant les saisons froides, lors du transport des appareils dans des moyens de transport insuffisamment ou non chauffés. Dans ces conditions, divers types de dommages ont été observés : dans la plupart des cas, une ondulation des membranes semi-perméables, due à leur allongement, associée à :

- une diminution importante de la perméabilité hydraulique pouvant être égale ou supérieure à 20 % de la perméabilité hydraulique initiale, ou

- une perte d'intégrité de l'appareil résultant de l'apparition de trous dans les membranes semi-perméables et/ou des décollements à l'interface entre les membranes semi-perméables et les masses d'empotage et/ou des fissurations dans les masses d'empotage, ces dommages conduisant généralement à des fuites de liquide entre les deux compartiments.

Afin d'éviter l'apparition de dommages dans les appareils comprenant des membranes semi-perméables humides, lorsqu'ils sont exposés à des températures inférieures à 0° C, et également afin d'empêcher la croissance des bactéries et des moisissures, il a été proposé, dans la demande de brevet japonais n° 6296838 (KOKAI), de remplir les appareils ou de mouiller la membrane semi-perméable à l'aide d'une solution aqueuse contenant de 20 à 80% en poids de glycérol (de préférence de 30 à 70%) et contenant de 5 à 40% en poids d'un alcool aliphatique.

Les alcools aliphatiques recommandés dans cette demande sont le méthanol, l'éthanol, le propanol et l'isopropanol, qui présentent l'inconvénient d'être très inflammables. Mais, les appareils ainsi traités ne peuvent pas être stérilisés par irradiation gamma car cette stérilisation énergétique conduit à la transformation des alcools aliphatiques précités en produits tels que des aldéhydes, qui sont toxiques aux concentrations atteintes après cette transformation.

La solution proposée dans cette demande n'est, en conséquence, pas souhaitable dans les applications médicales.

En outre, la demande de brevet japonais n° 6296838 enseigne qu'une concentration en glycérol inférieure à 20% en poids dans la solution aqueuse revendiquée ne permet pas d'empêcher la congélation de cette solution.

Il est connue (US-A-4609728) de fabriquer des membranes semi-perméables sèches, imprégnées de glycérol, afin de les protéger des radiations lors de la stérilisation par irradiation gamma, et d'éviter des pertes de perméabilité hydraulique. Bien entendu, il n'est pas constaté, dans ce cas, de dommages pour ces membranes lorsqu'elles sont exposées à des températures inférieures à 0° C, du fait que la quantité d'eau qu'elles renferment est faible ou nulle.

Il est également connu de conserver à l'état humide certaines membranes semi-perméables en les imprégnant de solutions aqueuses concentrées en glycérol (au moins 40% en poids de glycérol) car sinon elles perdraient irréversiblement leur perméabilité hydraulique ainsi que leurs caractéristiques mécaniques et deviendraient, en conséquence, inutilisables dans les applications médicales. Parmi les membranes semi-perméables conservées à l'état humide, on peut citer celles qui sont en polyacrylonitrile.

De façon classique, avant utilisation, les appareils pour le traitement extracorporel du sang ou du plasma sont dégazés et rincés avec une solution aqueuse de chlorure de sodium, stérile et apyrogène. Dans le cas des appareils comprenant des membranes semi-perméables sèches et imprégnées de glycérol ou des membranes semi-perméables humides imprégnées de solutions aqueuses concentrées de glycérol, il est recommandé de prolonger l'étape de rinçage pour éliminer les bulles d'air emprisonnées dans le canal interne des membranes ayant la forme d'un faisceau de fibres. En outre, en raison de fortes teneurs de glycérol dans les membranes, il peut être parfois nécessaire de tapoter les appareils pour favoriser le dégazage.

Le glycérol est en outre connu pour ses propriétés d'antigel. Mais, des concentrations élevées de glycérol sont nécessaires pour abaisser le point de congélation de l'eau de quelques degrés (Celsius), comme on peut le constater à la lecture du tableau qui suit, dans lequel on a reporté la température de congélation d'une solution aqueuse de glycérol en fonction de la quantité de glycérol dans la solution. Ces données chiffrées sont extraites de l'ouvrage intitulé : "Handbook of Chemistry and Physics" 65ième édition, CRC Press, 1975, page D-235

| % de glycérol dans la solution aqueuse (en poids) | abaissement de la température de congélation de l'eau (en °Celsius) |
|---|---|
| 0,50 | 0,072 |
| 1,00 | 0,180 |
| 2,00 | 0,411 |
| 3,00 | 0,627 |
| 4,00 | 0,849 |
| 5,00 | 1,078 |
| 6,00 | 1,316 |
| 7,00 | 1,561 |
| 8,00 | 1,811 |
| 9,00 | 2,064 |
| 10,00 | 2,323 |
| 12,00 | 2,880 |
| 14,00 | 3,469 |
| 16,00 | 4,094 |
| 18,00 | 4,756 |
| 20,00 | 5,46 |
| 24,00 | 7,01 |
| 28,00 | 8,77 |
| 32,00 | 10,74 |
| 36,00 | 12,96 |
| 40,00 | 15,50 |

De manière surprenante, le déposant a trouvé qu'il est possible de limiter les risques de fuite et les variations de la perméabilité hydraulique d'un appareil pour le traitement extracorporel de sang ou de plasma comprenant deux compartiments, un compartiment destiné à la circulation du sang ou du plasma, et un compartiment destiné à la circulation de liquide usé, séparés par une membrane semi-perméable humide, lorsque cet appareil est soumis à une température inférieure à 0° C, par exemple - 18° C, pendant une période de temps variable pouvant aller de un jour à plus d'un mois, si cet appareil présente, avant et après stérilisation, les trois caractéristiques techniques qui suivent :

- la membrane est imprégnée d'une solution aqueuse de glycérol ;

- la solution aqueuse de glycérol contient de 7 à 15% en poids de glycérol et est exempte de composés chimiques toxiques ;

- les deux compartiments sont purgés de la solution aqueuse de glycérol.

La solution mise au point dans le cadre de la présente invention pour améliorer la résistance au froid (température inférieure à 0° C, par exemple égale à - 18° C) des appareils pour le traitement extracorporel du sang ou du plasma comprenant une membrane semi-perméable humide, conduit à un résultat tout à fait surprenant à la lumière des connaissances apportées par l'état de la technique précité.

En effet, la solution aqueuse selon la présente invention, utilisée pour imprégner la membrane semi-perméable, contient une faible quantité de glycérol, de 7 à 15% en poids rapporté au poids total de la solution, de préférence de 8 à 12% en poids, ces quantités étant estimées insuffisantes par l'état de la technique précité, en particulier la demande de brevet japonais n° 6296838 (KOKAI) et les données précitées apparaissant dans l'ouvrage intitulé "Handbook of Chemistry and Physics".

Grâce à l'invention, les températures auxquelles un appareil peut être soumis sans apparition de fuites entre les compartiments ou variation importante de la perméabilité hydraulique peuvent atteindre - 20°C, voire être un peu inférieures à - 20°C, lorsqu'on utilise une solution aqueuse de glycérol contenant de 10 à 15% en poids de glycérol.

L'invention présente, en outre, plusieurs avantages majeurs : premièrement, la membrane imprégnée de la solution aqueuse de glycérol contenant de 7 à 15% en poids de glycérol n'est pas endommagée par une stérilisation très énergétique, comme la stérilisation par irradiation gamma ; deuxièmement, la mise en oeuvre de l'appareil par l'utilisateur est exactement identique à celle de tout appareil du même type, l'appareil étant, de plus, facile à manipuler du fait que ses deux compartiments sont purgés, et facile à rincer avant utilisation, comparativement à un appareil du même type comprenant une membrane glycérinée (au moins 40 % en poids de glycérol rapporté au poids de la membrane) ; troisièmement, les caractéristiques (hémocompatibilité, capacité de transfert diffusif et convectif, capacité d'adsorption protéique) de l'appareil soumis à une température inférieure à 0°C, par exemple égale à -10°C ou - 18°C, pendant une période de temps variable pouvant aller de un jour à plus d'un mois, ne sont pas altérées de façon significative si on les compare à un même appareil conservé à une température ambiante supérieure à 0°C.

Avantageusement, la quantité minimale de solution aqueuse de glycérol représente 50% en poids rapporté au poids total de la membrane semi-perméable.

Conformément à l'invention, la solution aqueuse de glycérol est exempte de composés chimiques qui sont toxiques ou qui deviennent toxiques après stérilisation énergétique, du type irradiation gamma. En particulier, la solution aqueuse de glycérol est exempte d'alcools aliphatiques monohydriques, comme le méthanol, l'éthanol, le propanol et l'isopropanol. Egalement, la solution aqueuse de glycérol est exempte de composés chimiques toxiques mais connus pour leurs propriétés d'antigel, comme par exemple l'éthylène glycol.

En revanche, la solution aqueuse de glycérol peut comprendre un ou plusieurs composés chimiques destinés à traiter la membrane semi-perméable dans la masse ou en surface pour améliorer sa biocompatibilité : à titre d'exemple, on peut citer les polyéthylèneimines (PEI), les polyvinylpyrrolidones (PVP) et les polyéthylèneglycols (PEG).

La nature chimique de la membrane semi-perméable de l'appareil selon l'invention n'est pas critique. Elle peut être à base, par exemple, de polyacrylonitrile, de polyméthacrylate de méthyle, de polysulfone, de polyéthersulfone, de cellulose, de polyamide.

La présente invention convient plus particulièrement aux appareils comprenant une membrane semi-perméable qui doit être conservée à l'état humide, comme les membranes en polyacrylonitrile ou les membranes en polyméthacrylate de méthyle.

Avantageusement, la membrane semi-perméable est une membrane plane ou un faisceau de fibres creuses constitué(e) d'au moins un polymère qui est un homo- ou un co-polymère d'acrylonitrile, ce polymère étant de préférence électronégatif. A titre d'exemple de copolymère d'acrylonitrile, convenant à la présente invention, on peut citer :

1) un copolymère d'acrylonitrile et d'au moins un monomère anionique ou anionisable, renfermant, le cas échéant, des unités provenant d'au moins un autre monomère à insaturation oléfinique capable d'être copolymérisé avec l'acrylonitrile, ou

2) un copolymère d'acrylonitrile et d'au moins un monomère anionique ou anionisable et d'au moins un monomère non ionique et non ionisable.

Certains de ces composés macromoléculaires, ainsi que les divers monomères susceptibles d'être retenus comme matières premières et leur fabrication, sont décrits dans le brevet américain n° 4,545,910 redélivré sous le n° Re.34239.

Parmi ces composés macromoléculaires, ceux avec lesquels l'appareil médical selon l'invention est particulièrement bien adapté sont définis plus haut sous (1). En particulier, l'invention convient particulièrement bien aux composés pour lesquels le comonomère anionique ou anionisable est oléfiniquement insaturé et porteur de groupements anioniques choisis parmi les groupes sulfonate, carboxyle, phosphate, phosphonate et sulfate, et plus particulièrement encore, quand ce comonomère est le méthallylsulfonate de sodium : cette membrane, qui est fabriquée par la société HOSPAL et connue sous le nom commercial AN69, doit être conservée à l'état humide, et contient généralement de l'ordre de 70% en poids d'eau.

Bien entendu, la nature précise du contre-ion des groupements anioniques n'est pas essentielle au bon fonctionnement de l'invention.

Parmi les monomères à insaturation oléfinique capables d'être copolymérisés avec l'acrylonitrile, on peut citer les acrylates d'alkyle et, en particulier, l'acrylate de méthyle.

La présente invention convient également aux appareils comprenant une membrane semi-perméable traitée, dans la masse ou en surface, pour en améliorer la biocompatibilité [de façon que les réactions (coagulation, notamment) qui se produisent lorsque le sang entre au contact d'un matériau étranger ne se produisent pas ou à des niveaux relativement bénins].

A titre d'exemple, on peut citer :

- l'appareil décrit dans la demande de brevet européen n° 0 801 953 qui comprend une membrane semi-perméable constituée d'au moins un polymère électronégatif, en particulier un polyacrylonitrile du type de ceux décrits ci-dessus, traitée dans la masse avec un agent cationique et antiprotease, de préférence le nafamostat mésilate ;

- l'appareil décrit dans la demande de brevet européen n° 0 925 826 qui comprend une membrane semi-perméable, à base de polyacrylonitrile porteur de charges négatives fixes, traitée avec un polymère neutre tel que les polyvinylpyrrolidones (PVP) et les polyéthylèneglycols (PEG), ou avec un polymère cationique, tel que les polyéthylèneimines (PEI).

L'invention a pour objet un procédé pour limiter les risques de fuite et les variations de la perméabilité hydraulique d'un appareil pour le traitement extracorporel de sang ou de plasma qui est soumis à une température inférieure à 0° C, cet appareil comprenant deux compartiments, un compartiment destiné à la circulation du sang ou du plasma, et un compartiment destiné à la circulation de liquide usé, séparés par une membrane semi-perméable humide, le procédé comprenant les étapes de :

- préparer une solution aqueuse de glycérol contenant de 7 à 15% en poids de glycérol et exempte de composés chimiques toxiques avant ou après stérilisation énergétique, du type irradiation gamma ;

- porter la solution aqueuse de glycérol au contact de la membrane semi-perméable ;

- purger l'appareil de la solution aqueuse de glycérol ;

- stériliser l'appareil.

Selon des variantes d'exécution du procédé selon l'invention, on porte la solution aqueuse de glycérol au contact de la membrane semi-perméable en faisant circuler cette solution :

- dans le compartiment destiné à la circulation du sang ou du plasma,

- ou, dans le compartiment destiné à la circulation du liquide usé,

- ou, dans le compartiment destiné à la circulation du sang ou du plasma et dans le compartiment destiné à la circulation du liquide usé.

Selon d'autres variantes d'exécution du procédé selon l'invention :

- la solution aqueuse de glycérol contient de 8 à 12% en poids de glycérol,

- et/ou, la solution aqueuse de glycérol contient un ou plusieurs composés chimiques destinés à traiter la membrane semi-perméable dans la masse ou en surface pour améliorer sa biocompatibilité, de préférence un composé chimique choisi parmi les polyéthylèneimines (PEI) ; s'agissant de la quantité de ces composés chimiques à prévoir dans la solution aqueuse de glycérol, on pourra, par exemple, se reporter aux conditions opératoires énoncées dans la demande de brevet européen n° 0 925 826.

Dans le cas où la membrane est traitée dans la masse ou en surface pour améliorer sa biocompatibilité, le procédé selon l'invention peut être mis en oeuvre avec les étapes de :

- préparer une solution contenant un ou plusieurs composés chimiques destinés à améliorer la biocompatibilité de la membrane ;
- porter cette solution au contact de la surface de la membrane destinée à être mise au contact avec le sang ou le plasma ;
- assembler les divers composants de l'appareil, en particulier monter la membrane dans un boîtier et réaliser les embouts de ce boîtier, si cet assemblage n'a pas été réalisé avant les deux premières étapes précitées ;
- préparer une solution aqueuse de glycérol contenant de 7 à 15% en poids de glycérol et exempte de composés chimiques toxiques avant ou après stérilisation énergétique, du type irradiation gamma ;
- porter la solution aqueuse de glycérol au contact de la membrane semi-perméable ;
- purger l'appareil de la solution aqueuse de glycérol ;
- stériliser l'appareil.

Si nécessaire, avant de porter la solution aqueuse de glycérol au contact de la membrane semi-perméable, on rince la membrane avec de l'eau ou une solution aqueuse, par exemple une solution aqueuse de chlorure de sodium, afin d'éliminer certain(s) composé(s) chimique(s) présent(s) temporairement dans la membrane, qui sont utiles pour sa fabrication et/ou sa conservation. C'est le cas, par exemple, de la membrane AN69 dont la fabrication et la conservation font intervenir l'utilisation de glycérol : en pratique, la membrane AN69 est conservée à l'état humide et, pour ce faire, est glycérinée par trempage dans un mélange eau/glycérol, le plus souvent dans des proportion pondérales correspondantes 40/60. Dans le cadre de la présente invention, la membrane doit par conséquent être déglycérinée avant d'être imprégnée de la solution aqueuse glycérol contenant de 7 à 15% en poids de glycérol. L'opération de rinçage afin de déglycériner la membrane AN69 est réalisée en portant de l'eau ou une solution aqueuse, par exemple une solution aqueuse de chlorure de sodium au contact de la membrane AN69. Dans le cas où la membrane AN69 est sous la forme d'un faisceau de fibres creuses, l'opération de rinçage est réalisée, de préférence, en faisant circuler de l'eau ou une solution aqueuse de chlorure de sodium dans le compartiment destiné à la circulation du sang ou du plasma.

D'autres caractéristiques et avantages de l'invention apparaîtront à la lecture des exemples qui suivent. On se reportera également aux dessins annexés sur lesquels :

- la figure 1 représente une vue en coupe longitudinale schématique d'un dialyseur à fibres creuses ;

- la figure 2 représente l'effet de la quantité de glycérol dans la solution aqueuse utilisée pour imprégner de la membrane AN69 sur l'apparition de fuites entre les compartiments de dialyseurs stockés à - 10° C ;

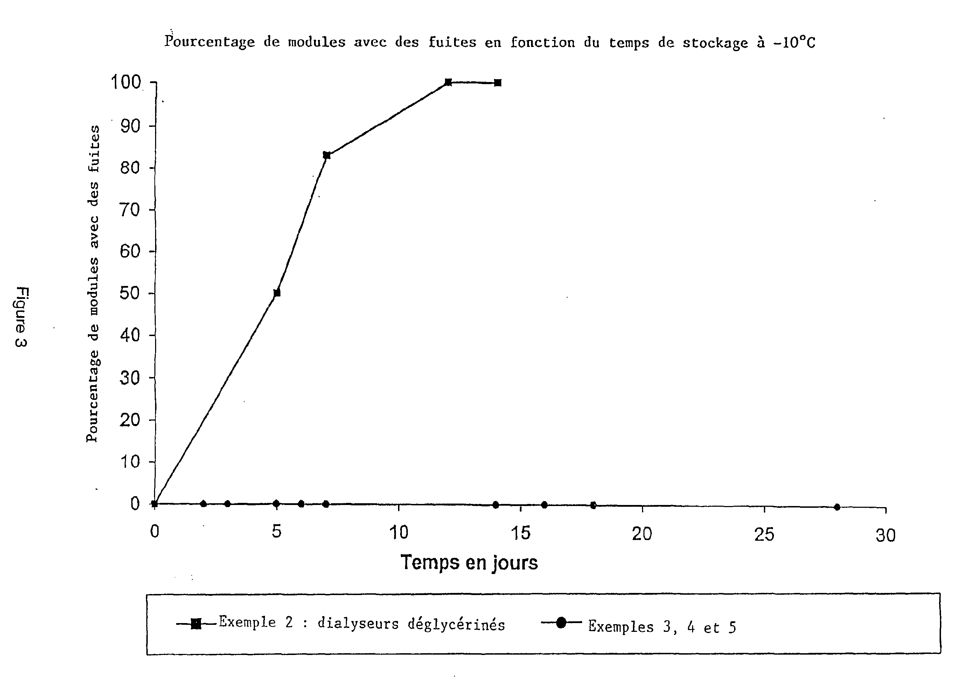

- la figure 3 représente l'effet de la quantité de glycérol dans la solution aqueuse utilisée pour imprégner de la membrane AN69 sur la perméabilité hydraulique de dialyseurs stockés à -10° C

## **EXEMPLES**

Pour illustrer l'invention, on a utilisé un type particulier d'appareil pour le traitement extracorporel du sang qui est utilisé pour pallier l'insuffisance rénale.

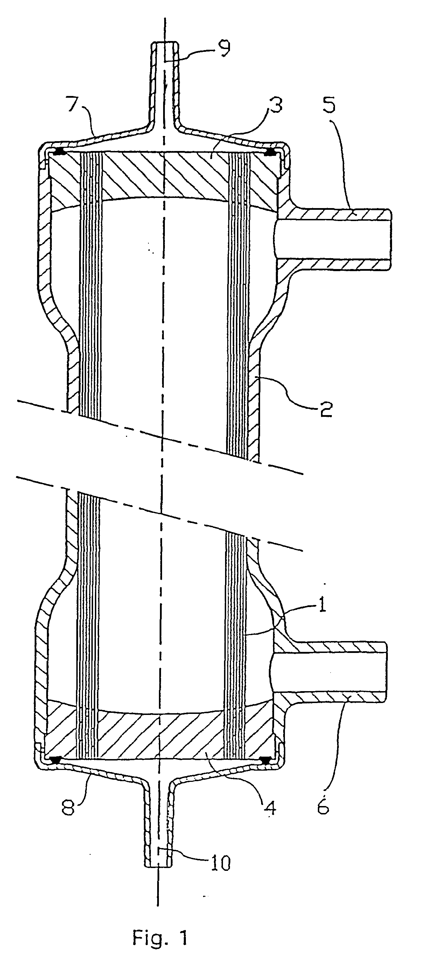

Le type d'appareil utilisé dans les exemples est un hémodialyseur/hémofiltre comprenant, de façon classique deux compartiments séparés par une membrane semi-perméable. Un premier compartiment est destiné à être relié au moyen d'une canalisation de prélèvement et d'une canalisation de restitution au circuit vasculaire du patient, tandis que le second compartiment a une entrée éventuellement reliée à une source de liquide de dialyse (traitement par hémodialyse et hémodiafiltration) et une sortie reliée à une évacuation de liquide usé (dialysat usé et/ou ultrafiltrat). La membrane est choisie pour permettre les transferts diffusifs et/ou convectifs des déchets du métabolisme, à partir du compartiment sang vers le compartiment pour liquide usé. La membrane peut être fabriquée sous la forme d'une membrane plane ou d'un faisceau de fibres creuses. Un dialyseur à membrane plane comprend une bande de membrane plane pliée en accordéon, une plaque intercalaire étant introduite dans tous les plis ouvrant d'un même côté. Comme on peut le voir sur la figure 1, un dialyseur à fibres creuses comprend un faisceau de fibres creuses 1, qui est disposé dans un boîtier tubulaire 2 où il est assujetti à ses deux extrémités par un disque de colle 3, 4. Outre de lier les fibres les unes aux autres, les disques de colle 3, 4 ont pour fonction de délimiter dans le boîtier tubulaire 2 un compartiment étanche auquel deux tubulures 5, 6, perpendiculaires à l'axe du boîtier 2, donnent accès. A chaque extrémité du boîtier 2 est fixé un embout 7, 8 comprenant une tubulure d'accès axiale 9, 10. Les deux tubulures 9, 10 sont symétriques. Le compartiment sang de cet appareil est constitué par l'espace intérieur délimité entre chaque disque de colle 3, 4 et l'embout 8, 9 fermant l'extrémité correspondante du boîtier tubulaire 2, et par l'intérieur des fibres creuses.

La membrane semi-perméable des dialyseurs exemplifiés est une membrane AN69 sous la forme d'un faisceau de fibres creuses.

On rappelle brièvement les principales étapes de la fabrication d'une fibre creuse en AN69. Un mélange de polymère est préparé, contenant 35% en poids d'un copolymère d'acrylonitrile et de méthallyle sulfonate de sodium, 52% en poids de diméthylformamide (DMF) et 13% en poids de glycérol. Le mélange de polymère est chauffé à 130° C et est extrudé dans une filière ayant deux buses concentriques, de l'azote étant injecté dans la buse interne pour former la lumière de la fibre creuse. Au contact de l'air ambiant (environ 20-25° C), la fibre de gel thermoréversible sortant de la filière est le siège d'une inversion de phase thermique. La fibre est ensuite reçue dans un bain d'eau dans lequel le solvant (DMF) dans la fibre est remplacé par de l'eau. La fibre est ensuite plongée dans de l'eau chaude à 95° C où elle est étirée de l'ordre de quatre fois. Suit une phase de stabilisation dans de l'eau chaude à 95 °C. Enfin, la fibre est glycérinée avec un mélange eau/glycérol, selon les proportions pondérales 40/60.

Dans les exemples qui suivent, les dialyseurs testés sont des dialyseurs (nom commercial NEPHRAL 300, fabriqué par HOSPAL INDUSTRIE, France), équipés d'un faisceau de fibres creuses AN69 de surface utile de 1,3 $m^2$.

Conformément à l'invention, pour limiter les risques de fuite et les variations de la perméabilité hydraulique de ces dialyseurs lorsqu'ils sont soumis à une température inférieure à 0°C, pouvant atteindre - 20°C, on imprègne la membrane semi-perméable avec une solution d'eau déminéralisée et de glycérol contenant de 7 à 15% en poids de glycérol, cette solution étant exempte de composés chimiques toxiques ou susceptibles de le devenir après une stérilisation énergétique comme la stérilisation par irradiation gamma. On réalise cette étape d'imprégnation après assemblage de l'appareil, comme représenté sur la figure 1, après élimination de la glycérine nécessaire à la fabrication de la membrane AN69 et avant stérilisation.

De préférence, on imprègne la membrane semi-perméable en faisant circuler la solution aqueuse de glycérol dans le compartiment sang : pour ce faire, on connecte l'une des tubulures 9 (10) du circuit sang à un récipient contenant la solution aqueuse de glycérol, on connecte l'autre tubulure d'accès 10 (9) à un récipient de recueil vide, et on provoque la circulation de la solution aqueuse de glycérol dans le compartiment sang, le cas échéant

Dans les exemples 3 à 6 ci-après, les conditions précises de l'imprégnation et des étape suivantes sont :

1) circulation dans le compartiment sang (comprenant l'intérieur des fibres) de 1 litre d'eau (débit de 200 ml/min) afin de rincer la membrane semi-perméable, en particulier afin d'éliminer la glycérine utile à la fabrication de la membrane ;

2) circulation dans le compartiment sang de 2 litres d'une solution de glycérol dans de l'eau déminéralisée contenant, selon le cas, 5, 10 ou 15% en masse de glycérol, à un débit de 250 ml/min ;

3) purge à l'air des compartiments des dialyseurs durant environ 30 secondes en imposant une pression d'air de $5.10^4$ Pa (0,5 bar) dans le compartiment destiné à la circulation de liquide usé et une pression d'air de $3.10^4$ Pa (0,3 bar) dans le compartiment destiné à la circulation du sang ;

4) fermeture étanche des tubulures d'accès 5, 6, 9, 10 avec des bouchons ;

5) stérilisation par irradiation gamma (25-36 kGy).

## Exemples 1 à 5

Evaluation de la perméabilité hydraulique et de l'étanchéité de dialyseurs NEPHRAL 300 en fonction du temps de stockage dans une enceinte régulée à température de - 10°C.

5 groupes comprenant chacun 10 dialyseurs NEPHRAL 300 ont été évalués dans les exemples 1 à 5. Le tableau qui suit indique les caractéristiques qui différencient chaque groupe de dialyseurs.

| Exemples | Caractéristiques particulières |
|---|---|
| 1 (témoin n° 1) | Les 10 dialyseurs NEPHRAL 300 sont déglycérinés, non traités avec une solution aqueuse de glycérol et stockés à une température ambiante de l'ordre de 20°C. |
| 2 (témoin n° 2) | Les 10 dialyseurs NEPHRAL 300 sont déglycérinés, non traités avec une solution aqueuse de glycérol et stockés à une température de - 10°C. |

(suite)

| Exemples | Caractéristiques particulières |
|----------|-------------------------------|
| 3 | Les 10 dialyseurs NEPHRAL 300 sont déglycérinés, traités avec une solution aqueuse contenant 5% en poids de glycérol et stockés à - 10°C. |
| 4 | Les 10 dialyseurs NEPHRAL 300 sont déglycérinés, traités avec une solution aqueuse contenant 10% en poids de glycérol et stockés à - 10°C. |
| 5 | Les 10 dialyseurs NEPHRAL 300 sont déglycérinés, traités avec une solution aqueuse contenant 15% en poids de glycérol et stockés à - 10°C. |

Dans les exemples 3, 4 et 5, la solution aqueuse de glycérol représente environ 70% en poids de la membrane.

Le pourcentage en poids de glycérol dans chaque membrane des dialyseurs des exemples 3, 4 et 5, est respectivement de l'ordre de 3,5%, 7% et 10,5%.

La perméabilité hydraulique des dialyseurs des exemples 1 à 5 a été mesurée après 7 jours, 14 jours et 28 jours de stockage, selon le cas, à - 10°C ou à température ambiante de l'ordre de 20°C (exemple 1 : témoin n° 1).

On rappelle que la perméabilité hydraulique décrit la quantité d'eau qui peut être ultrafiltrée au travers d'une membrane semi-perméable de surface active déterminée, avec une pression transmembranaire déterminée dans une période de temps déterminée. Les conditions de la mesure de la perméabilité hydraulique dans les exemples sont les suivantes :

-   débit d'eau dans le compartiment destiné à la circulation du sang ou du plasma : 300 ml/min ;

-   pression transmembranaire : 85 mmHg.

La moyenne des valeurs de perméabilités hydrauliques mesurées dans chaque groupe de dialyseurs a été normalisée en prenant pour base 100 % de la moyenne des valeurs de perméabilités hydrauliques initiales de chaque groupe.

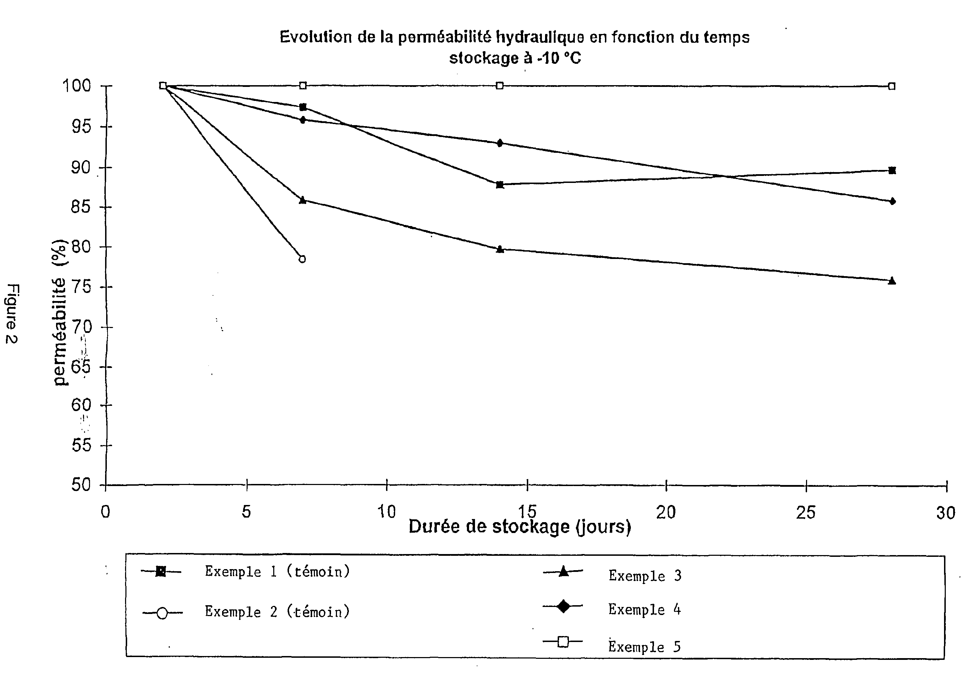

Les valeurs obtenues ont été reportées sur la figure 2 et permettent de connaître les pertes (en %) de perméabilité hydraulique.

Les dialyseurs de l'exemple 5 (traitement avec une solution aqueuse contenant 15% en poids de glycérol) sont d'une stabilité remarquable : pas de diminution de la perméabilité hydraulique après 28 jours à - 10°C.

Les dialyseurs de l'exemple 4 (traitement avec une solution aqueuse contenant 10% en poids de glycérol) ont un très bon comportement : la diminution de la perméabilité hydraulique après 28 jours à - 10°C n'est que de 15%, et est très proche de la diminution de perméabilité hydraulique des dialyseurs déglycérinés, stockés à température ambiante (exemple 1).

Les dialyseurs de l'exemple 3 (traitement avec une solution aqueuse contenant 5% en poids de glycérol) ne sont pas stables : la diminution de la perméabilité hydraulique est de 20% après 15 jours à - 10°C et de 25% environ après 28 jours à - 10°C.

Les dialyseurs de l'exemple 2 (déglycérinés et stockés à - 10°C) présentent tous des fuites après 7 jours à - 10°C : des observations macroscopiques et microscopiques de ces dialyseurs ont permis de mettre en évidence un allongement des fibres creuses, des fissures dans la colle d'empotage, des décollements fibres/colle et des trous dans les fibres.

### Exemple 6 - Stockage à - 18°C

10 dialyseurs de type NEPHRAL 300, déglycérinés, sont traités par circulation à l'intérieur des fibres de chaque dialyseur de 2 litres d'une solution contenant 10% de glycérol en masse (débit de 250 ml/min). Le liquide contenu dans le canal interne des fibres est purgé par circulation d'air. Les dialyseurs sont bouchés étanches et stérilisés par irradiation gamma. Les dialyseurs sont placés dans une enceinte régulée à la température de - 18°C. Après une semaine de stockage, les dialyseurs sont sortis de l'enceinte. L'étanchéité des 10 dialyseurs a été testée avec un appareil ATEQ permettant une mise en pression (sous 1 bar) du compartiment destiné à la circulation du sang ou du plasma, l'autre compartiment étant ouvert : on vérifie l'absence ou non de la chute de pression à la sortie du compartiment destiné à la circulation du sang ou du plasma. La perméabilité hydraulique et la clairance à l'urée et à la vitamine B12 ont été évaluées sur 3 dialyseurs de la série.

Les conditions opératoires des mesures de perméabilités hydrauliques sont identiques à celles des exemples 1 à 5.

Les conditions opératoires des mesures de clairance à l'urée et à la vitamine B12 sont les suivantes :

- préalablement, on rince le dialyseur avec 2 litres de sérum physiologique ;

- on fait circuler dans le compartiment pour liquide usé un bain de dialyse au débit de 500 ml/min en circuit ouvert. Le bain de dialyse est une solution aqueuse contenant (en mmol/l) : sodium : 135, potassium : 1.5, magnésium : 0.75, calcium : 1.75, chlorure : 106.5, acétate : 35 ;

- on fait circuler dans le compartiment sang un bain de dialyse contenant 1 g/l d'urée et 100 mg/l de vitamine B12, en circuit fermé. Le volume de bain de dialyse est égal à 2' litres et est maintenu constant par apport de solution au débit de 10 ml/min, afin de compenser l'ultrafiltration. le débit du bain de dialyse est égal à 200 ml/min ;

- on fixe l'ultrafiltration au débit de 10 ml/min ;

- au bout de 30 min, on détermine les concentrations en urée et en vitamine B12 à l'entrée et à la sortie du compartiment sang.

La clairance qui représente le niveau d'épuration du dialyseur s'exprime :

$$\mathtt{clairance = [(QES \times CES) - (QES \times CSS)]/ CES}$$

avec :

QES = débit à l'entrée du compartiment sang
CES = concentration à l'entrée du compartiment sang
CSS = concentration à la sortie du compartiment sang

| Dialyseur | Etanchéité | Perméabilité hydraulique ml/h m² mmHg | Clairance urée ml/min | Clairance B12 ml/min |
|---|---|---|---|---|
| 6a | Oui | 40,8 | 218 | 90 |
| 6b | Oui | 46,4 | 236 | 111 |
| 6c | Oui | 53,2 | 236 | 112 |
| 6d à 6j | Oui | nm* | nm | nm |
| * nm signifie non mesuré | | | | |

Sachant que :

- la perméabilité des dialyseurs de l'exemple 6 doit être supérieure à 35,4 ml/h m² mmHg,
- les valeurs initiales de clairance en urée des dialyseurs de l'exemple 6 varient de 210 à 256 ml/min,
- les valeurs initiales de clairance en vitamine B12 des dialyseurs de l'exemple 6 varient de 90 à 134 ml/min,

on observe le bon comportement de ces dialyseurs, après un stockage d'une semaine à - 18°C.

**Revendications**

1. Procédé pour limiter les risques de fuite et les variations de la perméabilité hydraulique d'un appareil pour le traitement extracorporel de sang ou de plasma soumis à une température inférieure à 0°C, cet appareil comprenant deux compartiments, un compartiment destiné à la circulation du sang ou du plasma, et un compartiment destiné à la circulation de liquide usé, séparés par une membrane semi-perméable, ladite membrane semi-perméable étant une membrane qui doit être conservée à l'état humide, le procédé comprenant les étapes de :

- préparer une solution aqueuse de glycérol contenant de 7 à 15% en poids de glycérol et exempte de composés chimiques toxiques avant ou après stérilisation énergétique ;
- porter la solution aqueuse de glycérol au contact de la membrane semi-perméable ;
- purger l'appareil de la solution aqueuse de glycérol avant stérilisation:
- stériliser l'appareil par stérilisation énergétique.

2. Procédé selon la revendication 1 où la stérilisation énergétique est du type irradiation gamma.

3. Procédé selon la revendication 1 ou 2, **caractérisé en ce que** la solution aqueuse de glycérol contient de 8 à 12 % en poids de glycérol.

4. Procédé selon la revendication 1 ou 2, **caractérisé en ce que** la solution aqueuse de glycérol est exempte d'alcools aliphatiquesmonohydriques.

5. Procédé selon la revendication 1 ou 2, **caractérisé en ce que** l'on porte la solution aqueuse de glycérol au contact de la membrane semi-perméable en la faisant circuler dans le compartiment destiné à la circulation du sang ou du plasma.

6. Procédé selon la revendication 1 ou 2, **caractérisé en ce que** l'on porte la solution aqueuse de glycérol au contact de la membrane semi-perméable en la faisant circuler dans le compartiment destiné à la circulation du liquide usé.

7. Procédé selon la revendication 1 ou 2, **caractérisé en ce que** l'on porte la solution aqueuse de glycérol au contact de la membrane semi-perméable en la faisant circuler dans le compartiment destiné à la circulation du sang ou du plasma et dans le compartiment destiné à la circulation du liquide usé.

8. Procédé selon la revendication 1, comprenant en plus les étapes de :

- préparer une solution contenant un ou plusieurs composés chimiques destinés à améliorer la biocompatibilité de la membrane :
- porter cette solutions au contact de la surface de la membrane destinée à être mise au contact avec le sang ou le plasma ;
- monter la membrane dans un boîtier et réaliser les embouts de ce boîtier.

9. Procédé selon la revendication 8, où la stérilisation énergétique est du type irradiation gamma.

10. Procédé selon la revendication 8, **caractérisé en ce que** la solution aqueuse de glycérol contient de 8 à 12 % en poids de glycérol.

11. Procédé selon la revendication 8, **caractérisé en ce que** la solution aqueuse de glycérol est exempte d'alcools aliphatiquesmonohydriques.

12. Utilisation d'une solution aqueuse contenant de 7 à 15% en poids de glycérol pour imprégner les membranes semi-perméables humides avant stérilisation énergétique, afin de limiter la variation de perméabilité hydraulique de ces membranes lorsqu'elles sont soumises à une température inférieure à 0°C, pouvant atteindre -20°C.

**Claims**

1. A method for limiting leakage risks and variations of water permeability of a device for extracorporeal blood or plasma treatment subjected to a temperature below 0°C, this device comprising two compartments, one compartment intended for blood or plasma circulation, and one compartment intended for waste liquid circulation, separated by a semi-permeable membrane, said semi-permeable membrane being a membrane which must be stored in wet conditions, the method comprising the following steps:

- prepare an aqueous solution of glycerol containing 7 to 15% by weight of glycerol and free of toxic chemical compounds before or after energetic sterilization;
- contact the aqueous solution of glycerol with the semi-permeable membrane;
- purge the device of the aqueous solution of glycerol before sterilization;

- sterilize the device through energetic sterilization.

2. The method according to claim 1, wherein the energetic sterilization is of the gamma irradiation type.

3. The method according to claim 1 or 2, **characterized in that** the aqueous solution of glycerol contains 8 to 12% by weight of glycerol.

4. The method according to claim 1 or 2, **characterized in that** the aqueous solution of glycerol is free of monohydric aliphatic alcohols.

5. The method according to claim 1 or 2, **characterized in that** the aqueous solution of glycerol is contacted with the semi-permeable membrane by circulating the solution in the compartment intended for blood or plasma circulation.

6. The method according to claim 1 or 2, **characterized in that** the aqueous solution of glycerol is contacted with the semi-permeable membrane by circulating the solution in the compartment intended for waste liquid circulation.

7. The method according to claim 1 or 2, **characterized in that** the aqueous solution of glycerol is contacted with the semi-permeable membrane by circulating the solution in the compartment intended for blood or plasma circulation and in the compartment intended for waste liquid circulation.

8. The method according to claim 1, further comprising the following steps:

   - prepare a solution containing one or more chemical compounds apt to improve membrane biocompatibility;
   - contact this solution with the surface of the membrane to be contacted with blood or plasma;
   - place the membrane in a housing and carry out the connections of the housing.

9. The method according to claim 8, wherein the energetic sterilization is of the gamma irradiation type.

10. The method according to claim 8, **characterized in that** the aqueous solution of glycerol contains 8 to 12% by weight of glycerol.

11. The method according to claim 8, **characterized in that** the aqueous solution of glycerol is free of monohydric aliphatic alcohols.

12. Use of an aqueous solution containing 7 to 15% by weight of glycerol for impregnating wet semi-permeable membranes before the energetic sterilization, so as to limit the water permeability variation of these membranes when they are subjected to a temperature below 0°C, even up to -20°C.

**Patentansprüche**

1. Verfahren zur Begrenzung der Verlustgefahr und der Wasserpermeabilitätsänderungen von einer Vorrichtung zur extrakorporellen Blut- oder Plasmabehandlung, die einer Temperatur unter 0°C ausgesetzt ist, wobei diese Vorrichtung zwei Abteile umfasst, wobei ein Abteil zur Blut- oder Plasmazirkulation vorgesehen ist, und ein Abteil zur Zirkulation von gebrauchter Flüssigkeit vorgesehen ist, die durch eine semipermeable Membran voneinander getrennt sind, wobei die benannte semipermeable Membran eine Membran ist, die im nassen Zustand gelagert werden soll, wobei das Verfahren die folgenden Schritte umfasst:

   - Vorbereiten einer wässrigen Lösung aus Glyzerin, enthaltend 7 bis 15 Gew.-% Glyzerin und ohne toxische chemische Verbindungen vor oder nach Strahlungsfluss Sterilisation;
   - Kontaktieren der wässrigen Lösung aus Glyzerin mit der semipermeablen Membran;
   - Spülen der Vorrichtung von der wässrigen Lösung aus Glyzerin vor der Sterilisation;
   - Sterilisieren der Vorrichtung durch Strahlungsfluss Sterilisation.

2. Verfahren nach Anspruch 1, worin die Strahlungsfluss Sterilisation des Typs Gamma-Bestrahlung ist.

3. Verfahren nach Anspruch 1 oder 2, **dadurch gekennzeichnet, daß** die wässrige Lösung aus Glyzerin 8 bis 12 Gew.-% Glyzerin enthält.

4.  Verfahren nach Anspruch 1 oder 2, **dadurch gekennzeichnet, daß** die wässrige Lösung aus Glyzerin frei von einwertigen aliphatischen Alkoholen ist.

5.  Verfahren nach Anspruch 1 oder 2, **dadurch gekennzeichnet, daß** die wässrige Lösung aus Glyzerin mit der semipermeable Membran kontaktiert wird, indem sie im Abteil zur Blut- oder Plasmazirkulation zirkuliert wird.

6.  Verfahren nach Anspruch 1 oder 2, **dadurch gekennzeichnet, daß** die wässrige Lösung aus Glyzerin mit der semipermeable Membran kontaktiert wird, indem sie im Abteil zur Zirkulation von gebrauchter Flüssigkeit zirkuliert wird.

7.  Verfahren nach Anspruch 1 oder 2, **dadurch gekennzeichnet, daß** die wässrige Lösung aus Glyzerin mit der semipermeable Membran kontaktiert wird, indem sie im Abteil zur Blut- oder Plasmazirkulation und im Abteil zur Zirkulation von gebrauchter Flüssigkeit zirkuliert wird.

8.  Verfahren nach Anspruch 1, weiter umfassend die folgenden Schritte:

    - Vorbereiten einer Lösung enthaltend eine oder mehrere chemische Verbindungen zur Verbesserung der Membranbioverträglichkeit;
    - Kontaktieren dieser Lösung mit der Oberfläche der Membran, die zum Kontaktieren mit dem Blut oder Plasma vorgesehen ist;
    - Einsetzen der Membran in einem Gehäuse und Herstellen der Anschlüsse dieses Gehäuses:

9.  Verfahren nach Anspruch 8, worin die Strahlungsfluss Sterilisation des Typs Gamma-Bestrahlung ist.

10. Verfahren nach Anspruch 8, **dadurch gekennzeichnet, daß** die wässrige Lösung aus Glyzerin 8 bis 12 Gew.-% Glyzerin enthält.

11. Verfahren nach Anspruch 8, **dadurch gekennzeichnet, daß** die wässrige Lösung aus Glyzerin frei von einwertigen aliphatischen Alkoholen ist.

12. Verwendung einer wässrigen, Lösung enthaltend 7 bis 15 Gew.-% Glyzerin zur Tränkung der nassen semipermeablen Membranen vor die Strahlungsfluss Sterilisation, um die Wasserpermeabilitätsänderung dieser Membranen zu begrenzen, wenn sie einer Temperatur unter 0°C, auch bis -20°C, ausgesetzt sind.

Fig. 1

Evolution de la perméabilité hydraulique en fonction du temps
stockage à -10 °C

Durée de stockage (jours)

perméabilité (%)

| | | |
|---|---|---|
| —■— | Exemple 1 (témoin) | —▲— Exemple 3 |
| —○— | Exemple 2 (témoin) | —◆— Exemple 4 |
| | | —□— Exemple 5 |

Figure 2

14

EP 1 339 481 B1

Figure 3

**EP 1 339 481 B1**

**RÉFÉRENCES CITÉES DANS LA DESCRIPTION**

**Documents brevets cités dans la description**

- JP 6296838KOKAI A
- JP 6296838 B
- US 4609728 A
- EP 0801953 A
- EP 0925826 A

**Littérature non-brevet citée dans la description**

- Handbook of Chemistry and Physics. CRC Press, 1975, D-235